# EUROPEAN PATENT SPECIFICATION

(11) **EP 4 088 712 B1**
(45) Date of publication and mention of the grant of the patent: **25.03.2026**
(21) Application number: 20898263.7
(22) Date of filing: 11.12.2020
(51) Int. Cl.: A61K 8/365, A61Q 19/00, A61Q 19/08, A61Q 17/00

(54) **COMPOSITION FOR STIMULATING AND INDUCING CHANGES IN ENVIRONMENT AROUND AGING SKIN CELLS**
ZUSAMMENSETZUNG ZUM STIMULIEREN UND HERBEIFÜHREN VON VERÄNDERUNGEN IN DER UMGEBUNG ALTERNDER HAUTZELLEN
COMPOSITION POUR STIMULER ET INDUIRE DES CHANGEMENTS DANS L'ENVIRONNEMENT AUTOUR DE CELLULES CUTANÉES SUJETTES AU VIEILLISSEMENT

(30) Priority: 12.12.2019 KR 20190165821
(43) Date of publication of application: 16.11.2022
(73) Proprietor: Amorepacific Corporation, Seoul 04386 (KR)
(72) Inventor: PARK, Phil June, Yongin-si Gyeonggi-do 17074 (KR); CHOI, Hyunjung, Yongin-si Gyeonggi-do 17074 (KR)
(74) Representative: Schön, Christoph
(86) International application number: PCT/KR2020/018144
(87) International publication number: WO 2021/118286

(56) References cited:
- EP-A1- 1 762 219
- WO-A2-2010/065567
- CN-A- 104 717 965
- KR-A- 20080 100 943
- KR-A- 20150 024 605
- KR-A- 20150 083 698
- KR-B1- 101 827 374

## Description

### [Technical Field]

The present disclosure relates to a composition for stimulating growth of only human-derived young skin cells.

### [Background Art]

Skin, which is the largest organ of the human body, functions to protect the body from external physical and chemical stimuli and to maintain homeostasis such as humidity in the body, body temperature, and the like. However, when aged or continuously exposed to external stimuli, skin aging is rapidly progressing such as damage to the skin and deterioration of physiological functions. Such skin aging is a natural phenomenon that inevitably occurs as we age, but since the factors for the skin aging are increased due to various environmental factors, although the demand for quality of life is increasing due to improvement of living standards, countermeasures against skin aging are urgently required.

Skin aging is affected by various factors such as genetic and environmental factors, which are largely divided into natural aging or intrinsic aging and extrinsic aging. The natural aging or intrinsic aging is an aging phenomenon that occurs over time according to genetic information, which is mainly observed in skin not exposed to sunlight and features fine wrinkles and a decrease in skin elasticity and thus is difficult to artificially control. Extrinsic aging is a phenomenon caused by external environmental factors, especially UV rays present in sunlight, and is also called photoaging, which is found in skin exposed to sunlight and features deep wrinkles, rough skin texture, pigmentation, a decrease in skin tone and skin elasticity, and thus is easy to artificially control. Accordingly, research on prevention of extrinsic aging has continued, and in particular, research on prevention of wrinkle formation due to the extrinsic photoaging proceeded by long-term UV exposure has drawn attention (Gilchrest B.A., J. Am. Acad. Dermatol., 1989:21:610-613).

Accordingly, the term aging used in a cosmetic composition up to now refers to all extrinsic aging, particularly, skin aging caused by extrinsic photoaging, and anti-aging refers to a process in which cells aged by the external environmental factors are removed (suppressed from aging) or recovered and become young again (cell rejuvenation). This concept of the anti-aging is based on what is secured through improving aging factors of individual cells. However, desired effects are not yet actually achieved in various applications including clinical fields. According to the recent trend of the Dermatological Society, research on pursuing changes in the overall environment rather than suppressing the aging or rejuvenating cells through the anti-aging mechanism of the individual cells, and in particular, changes around each individual are mentioned as being more important than the individual cells.

Accordingly, the present inventors are not limited by the definition of aging used in conventional cosmetic compositions but have developed a composition applicable to extensive aging (intrinsic aging and extrinsic aging) and changing the aged environment to a non-aged environment by promoting growth of young cells alone that have not been aged yet, while the aged cells are left in the aged environment, instead of the anti-aging or the cell rejuvenation. See also the disclosure of EP1762219A1 and WO2010/065567A2.

This completely goes beyond the conventional concept of anti-aging and is expected to have a completely different mechanism from the existing anti-aging mechanism (anti-aging mechanism or cell rejuvenation mechanism) by specifically increasing the number of young cells around, while leaving the aged cells as they are to change the aged environment into a non-aged environment.

### [Disclosure]

### [Technical Problem]

An embodiment provides a composition for changing an aged environment through a mechanism of increasing the number of young cells relatively more than the number of aged cells by specifically promoting growth of the young cells that have not yet aged, while maintaining the number of the aged cells in the already aged environment.

### [Technical Solution]

A cosmetic use of a cosmetically acceptable compound represented by Chemical Formula 1, or a salt thereof, for stimulating only the growth of human-derived skin cells that are not stained with SA-β-gal (senescence associated-beta galactosidase):

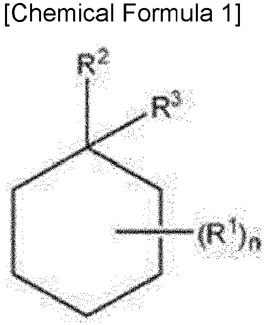

In Chemical Formula 1,
R¹ to R³ are each independently a hydroxy group or a carboxyl group, and
n is an integer of 1 to 3.

The compound represented by Chemical Formula 1 may be represented by Chemical Formula 1-1.

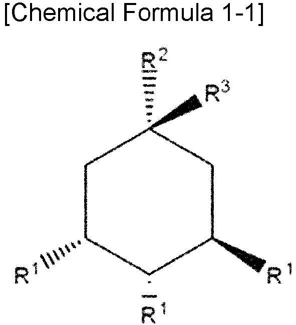

In Chemical Formula 1-1,
R¹ and R² are each independently a hydroxy group, and
R³ is a carboxyl group.

The compound represented by Chemical Formula 1 may be included in an amount of 0.01 wt% to 5 wt% based on the total amount of the composition.

The skin cells may be pigment-forming cells.

The cosmetic use may stimulate growth of only human-derived pigment-forming cells that are not stained with SA-β-gal (senescence associated-beta galactosidase) between human-derived pigment-forming cells that are stained with SA-β-gal (senescence associated-beta galactosidase) and human-derived pigment-forming cells that are not stained with SA-β-gal (senescence associated-beta galactosidase).

The skin cells may be fibroblasts.

The cosmetic use may stimulate a growth of only fibroblasts that are not stained with SA-β-gal (senescence associated-beta galactosidase) between human-derived fibroblasts that are stained with SA-β-gal (senescence associated-beta galactosidase) and human-derived fibroblasts (primary fibroblasts; FB) that are not stained with SA-β-gal (senescence associated-beta galactosidase).

The cosmetic use may increase the number of cells only in human-derived young skin cells that cannot be stained with SA-β-gal (senescence associated-beta galactosidase) and maintain the number of human-derived aged cells that are not stained with SA-β-gal (senescence associated-beta galactosidase).

The cosmetic use, wherein the compound is present in a cosmetic composition.

### [Advantageous Effects]

The cosmetic use according to an embodiment may improve an aged skin environment without removing aged cells or recovering the aged cells to young cells by applying a specific composition containing the compound represented by Chemical Formula 1 to skin to specifically grow the number of the young cells alone, while maintaining the number of the aged cells among the aged cells and non-aged young cells constituting aged skin.

### [Description of the Drawings]

FIG. 1 is an optical microscopic photograph showing the morphology and cell number of pigment-forming cells before and after UVB treatment.
FIG. 2 is an optical microscopic photograph of observation of changes in the morphology and number of UVB-treated pigment-forming cells according to an increase in the concentration of the compound represented by Chemical Formula E.
FIG. 3 is a graph showing ratios of the total number of pigment-forming cells, the number of aged pigment-forming cells, the number of SA-β-gal stained pigment-forming cells (aged cells), and the number of non-stained pigment-forming cells (young cells) by a concentration of the compound represented by Chemical Formula E.
FIG. 4 is an optical microscopic photograph showing the morphology and cell number of fibroblasts before and after UVB treatment.
FIG. 5 is an optical microscopic photograph of observation of changes in the morphology and cell number of UVB-treated fibroblasts according to an increase in the concentration of the compound represented by Chemical Formula E.
FIG. 6 is a graph showing ratios of the total number of fibroblasts, the number of aged fibroblasts, the number of SA-β-gal stained cells (aged cells), and the number of non-stained fibroblasts (young cells) by concentration of the compound represented by Chemical Formula E.

### [Mode for Invention]

Hereinafter, exemplary embodiments of the present disclosure will be described in detail, and may be easily performed by a person having ordinary skill in the related art.

In the present specification, aged cells refer to cells that are stained with SA-β-gal (senescence associated-beta galactosidase), and young cells refer to cells that are not stained with SA-β-gal (senescence associated-beta galactosidase).

In the present specification, it will be understood that when an element such as a layer, film, region, or substrate is referred to as being "on" another element, it may be directly on the other element or intervening elements may also be present. In contrast, when an element is referred to as being "directly on" another element, there are no intervening elements present.

In the present specification, when specific definition is not otherwise provided, "combination" refers to mixing or copolymerization. In addition, "copolymerization" refers to block copolymerization or random copolymerization, and "copolymer" refers to a block copolymer or a random copolymer.

Hereinafter, a composition for use according to claim 1 for stimulating growth of human-derived young skin cells that are not stained with SA-β-gal (senescence associated-beta galactosidase) according to an embodiment will be described.

A composition for stimulating induction of environmental changes around aged skin cells to stimulate only the growth of human-derived skin cells that are not stained with SA-β-gal (senescence associated-beta galactosidase) includes a compound represented by Chemical Formula 1, a derivative thereof, or a salt thereof that is cosmetically acceptable for cosmetics as an active ingredient, to skin.

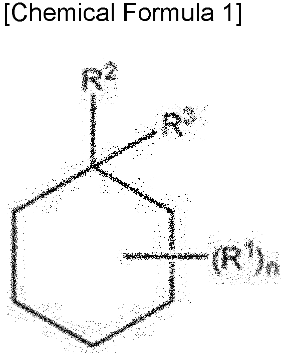

In Chemical Formula 1,
R¹ to R³ are each independently a hydroxy group or a carboxyl group, and
n is an integer of 1 to 3.

For example, the compound represented by Chemical Formula 1 may be represented by Chemical Formula 1-1.

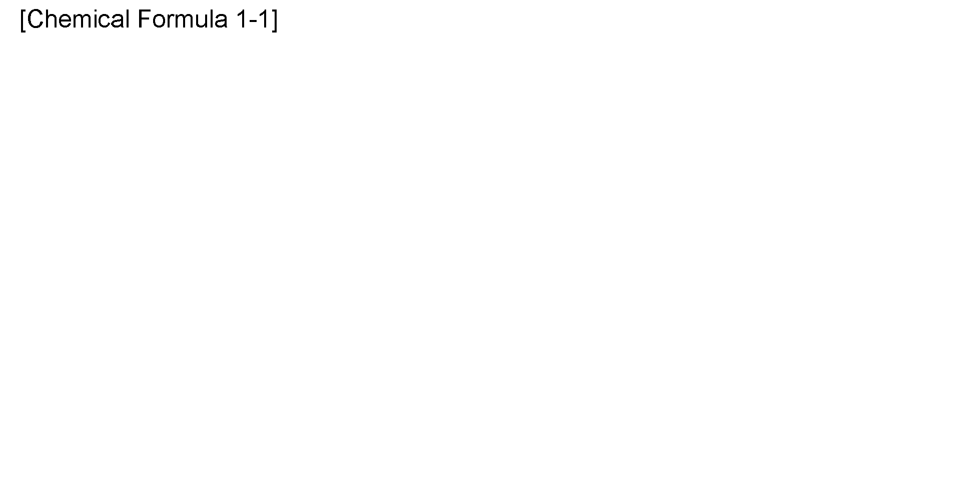

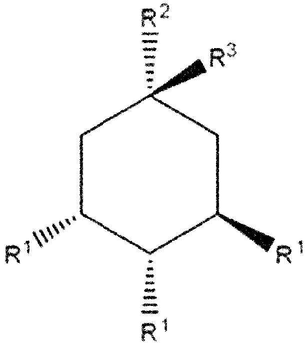

In Chemical Formula 1-1,
R¹ and R² are each independently a hydroxy group, and
R³ is a carboxyl group.

The present application, unlike conventional anti-aging technologies that focus on inhibiting aging of individual cells or improving individual aging factors, focuses on changes of the environment. The present application provides no recovery phenomenon in which cells aged by UV and the like are removed or rejuvenated but replaces the conventional anti-aging mechanism with a completely different mechanism therefrom in which growth of young cells (cells in which aging factors are not revealed) among the aged cells tends to increase in a concentration-dependent manner in a composition including the compound represented by Chemical Formula 1 and a derivative thereof or a salt thereof that is cosmetically acceptable for cosmetics as an active ingredient.

The compound represented by Chemical Formula 1 may selectively stimulate growth of only non-aged young pigment-forming cells between aged human-derived pigment-forming cells (primary melanocytes; MC) and non-aged human-derived pigment-forming cells (primary melanocytes; MC).

The compound represented by Chemical Formula 1 may selectively stimulate growth of only non-aged young fibroblasts between aged human-derived fibroblasts (FB) and non-aged human-derived fibroblasts (FB).

That is, the skin cells may be pigment-forming cells or fibroblasts.

The compound represented by Chemical Formula 1, a derivative thereof, or a salt thereof that is cosmetically acceptable for cosmetics may be included in an amount of 0.01 wt% to 5 wt% based on the total amount of the composition. When the compound represented by Chemical Formula 1, and the derivative thereof or salt thereof that is cosmetically acceptable for cosmetics is used in an amount of less than 0.01 wt%, the content is too low and thus an effect of stimulating the growth of young cells (cells in which the aging factor is not revealed) among aged cells is very insignificant, and when used in an amount of more than 5 wt%, it may be uneconomical because an effect of stimulating the growth of sufficiently young cells (cells in which the aging factor is not revealed) may be obtained even with an amount of 5 wt% or less.

That is, since the composition according to an embodiment includes the compound represented by Chemical Formula 1, a derivative thereof or a salt thereof that is cosmetically acceptable for cosmetics as an active ingredient, it increases the number of cells only in human-derived young skin cells that cannot be stained with SA-β-gal (senescence associated-beta galactosidase) and maintains the number of human-derived aged cells that are stained with SA-β-gal (senescence associated-beta galactosidase).

As used herein, the term "cosmetically effective amount" refers to an amount that is sufficient to exhibit the desired physiological or cosmetic activity when the physiologically active ingredient is administered to an animal or human. However, the cosmetically effective amount may be appropriately changed depending on severity of symptoms, ages, weights, health status, sex, administration route, and period of use of patients.

In addition, as used herein, "cosmetically acceptable" means that it is physiologically acceptable and does not normally cause allergic reactions such as skin troubles or dizziness or similar reactions when administered to humans.

On the other hand, the composition may include a carrier, an excipient, a diluent, and the like, and examples thereof may be lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinylpyrrolidone, water, methyl hydroxybenzoate, propyl hydroxy benzoate, talc, magnesium stearate, and mineral oils. In addition, it may further include fillers, anti-coagulants, lubricants, wetting agents, fragrances, emulsifiers, and antiseptics.

The composition is a cosmetic composition.

In the present specification "cosmetic" may refer to any material that may have a medical function in addition to the cosmetic function.

The formulation of the cosmetic composition is not particularly limited and may be appropriately selected as desired.

For example, the cosmetic composition may be formulated into formulations such as solutions, suspension liquids, emulsions, pastes, gels, creams, lotions, powders, soaps, surfactant-containing cleaners, oils, powder foundations, emulsion foundations, wax foundations, and sprays, but the present disclosure is not limited thereto. More specifically, it may be formulated into cosmetic compositions such as detergents, tonics, hair dressings, nourishing lotions, essences, serums, treatments, conditioners, shampoos, lotions, wools, or hair dyes, and the like, and may be formulated into basic cosmetics such as an oil-in-water (O/W) type, a water-in-oil (W/O), and the like. For example, the composition may have a formulation selected from skin lotion, skin toner, astringent, lotion, milk lotion, moisture lotion, nourishing lotion, massage cream, nourishing cream, moisture cream, hand cream, ointment, foundation, essence, nourishing essence, pack, soap, cleansing foam, cleansing lotion, cleansing cream, body lotion, body cleanser, lotion, ointment, gel, cream, patch, or spray. In addition, in the composition, in addition to the above-mentioned essential components in each formulation, other components may be appropriately selected and formulated without difficulty by a person of ordinary skill in the art according to types or use purposes of other external preparations. For example, ultraviolet (UV) blocking agents, hair conditioning agents, fragrances, and the like may be further included.

The cosmetic composition may include a cosmetically acceptable medium or base. These are all formulations that are suitable for topical applications. The cosmetic composition may be provided in the form of emulsions obtained by dispersing an oil phase in an aqueous phase, suspensions, microemulsions, microcapsules, microgranules, or ion-type (liposome) and/or non-ionized vesicle dispersing agents, or in the form of creams, skins, lotions, powders, ointments, sprays, or conceal sticks. These compositions may be prepared according to conventional methods in the art.

When the formulation of the present disclosure is a solution or emulsion, a solvent, a solubilizer, or an emulsifier may be used as carrier components. For example, water, ethanol, isopropanol, ethyl carbonate, ethyl acetate, benzyl alcohol, benzyl benzoate, propylene glycol, 1,3-butylglycol oil, glycerol aliphatic ester, polyethylene glycol, or fatty acid ester of sorbitan may be used.

If the formulation of the present disclosure is a suspension, the carrier component may be a diluent of a liquid such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, tragacanth, and the like.

If the formulation of the present disclosure is pastes, creams, or gels, the carrier component may be animal oil, vegetable oil, wax, paraffin, starch, tragacanth, cellulose derivatives, polyethylene glycol, silicone, bentonite, silica, talc, or zinc oxide.

If the formulation of the present disclosure is powders or sprays, the carrier component may be lactose, talc, silica, aluminum hydroxide, calcium silicate, or polyamide powders. Particularly, in the case of sprays, a propellant such as a chlorofluorohydrocarbon, propane/butane, or dimethyl ether may be additionally included.

In an embodiment of the present disclosure, the cosmetic composition may include thickeners. The thickeners included in the cosmetic composition of the present disclosure may be methyl cellulose, carboxyl methyl cellulose, carboxyl methyl hydroxy guanine, hydroxy methyl cellulose, hydroxyethyl cellulose, a carboxyl vinyl polymer, polyquaternium, cetearyl alcohol, stearic acid, and carrageenan, preferably one or more of carboxyl methyl cellulose, a carboxyl vinyl polymer, and polyquaternium may be used, and more preferably a carboxyl vinyl polymer may be used.

In an embodiment of the present disclosure, the cosmetic composition may include a variety of suitable bases and additives as needed, and the types and amounts of these components may be easily selected by the inventor. If necessary, it may include an acceptable additive, and may further include, for example, conventional ingredients such as antiseptics, pigments, additives, and the like.

The antiseptics may specifically be phenoxyethanol or 1,2-hexanediol, and the fragrances may be artificial fragrances.

In an embodiment of the present disclosure, the cosmetic composition may include a composition selected from a water-soluble vitamin, an oil-soluble vitamin, a polymeric peptide, a polymeric polysaccharide, a sphingolipid, and a seaweed extract. Other ingredients that may be added include fats and oils, humectants, emollients, surfactants, organic and inorganic pigments, organic powders, ultraviolet (UV) absorbers, antiseptics, fungicides, antioxidants, plant extracts, pH adjusters, alcohols, pigments, fragrances, blood circulation accelerators, coolants, anhidrotics, purified water, and the like.

In addition, the compounding components which may be added other than these are not limited thereto and any component may be blended in the range which does not damage the purpose and effect of the invention.

Furthermore, the cosmetic composition according to an embodiment may be used as a dietary supplement. For example, it may be easily used as main ingredients, auxiliary ingredients, food ingredients, food additives, functional foods, or beverages.

The "food" means a natural or processed product including one or more nutrients, and preferably means that it is ready to be eaten directly after a certain amount of processing. It includes all foods, food additives, functional foods, and beverages.

The foods to which the food composition can be added may include, for example, various foods, beverages, gums, teas, vitamin composites, and functional foods. In addition, the foods may include special nutritional products (e.g., formulas, baby food, etc.), processed meat products, fish products, tofu, jellies, noodles (e.g. ramen noodles, etc.), breads, dietary supplements, seasoned foods (e.g., soy sauce, soybean paste, red pepper paste, mixed soy sauce, etc.), sauces, sweets (e.g. snacks), candy, chocolate, gum, ice cream, dairy products (e.g. fermented milk, cheese, etc.), other processed foods, kimchi, pickles (various kimchis, pickles, etc.), beverages (e.g., fruit beverages, vegetable beverages, soy milk, fermented beverages, etc.), and natural seasonings (e.g., ramen soup, etc.), but are not limited thereto. The foods, beverages, or food additives may be prepared by conventional manufacturing methods.

In addition, "functional foods" or "health functional foods" refers to a food group that has added values to foods by using physical, biochemical, or biotechnological techniques to act and express functions of foods for specific purposes, or foods that are processed and designed to fully express the body's regulatory functions, such as defense rhythm control of food compositions, disease prevention, and recovery of living bodies and may specifically be health functional foods. The functional foods may include acceptable food auxiliary additives and may further include suitable carriers, excipients, and diluents commonly used in the manufacture of functional foods.

The types of dietary supplements are not limited thereto, but may be in a form of powders, granules, tablets, capsules, or beverages.

### DETAILED DESCRIPTION OF THE EMBODIMENTS

Advantages and features of the present disclosure and methods for achieving them will be apparent with reference to the examples described in detail below. The present disclosure will be described in detail with reference to examples. However, these examples are specifically provided for describing the present disclosure, and the range of the present disclosure is not limited to these examples.

### (Examples)

### Experimental Example 1: Change in morphology and cell number of pigment-forming cells by UVB treatment

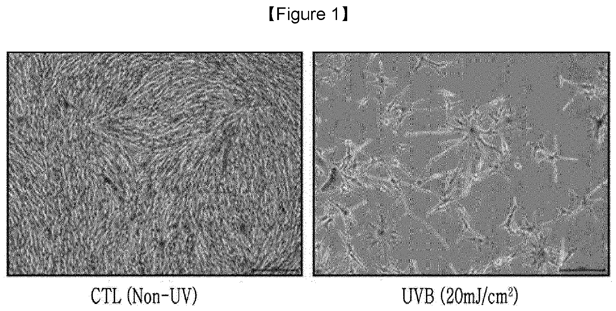

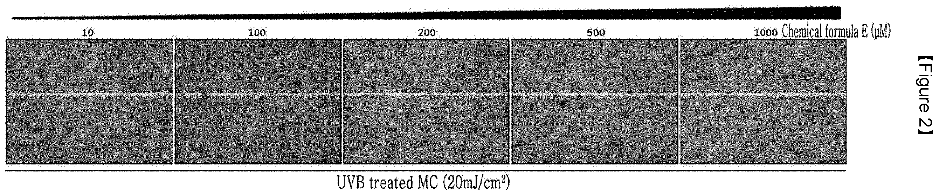

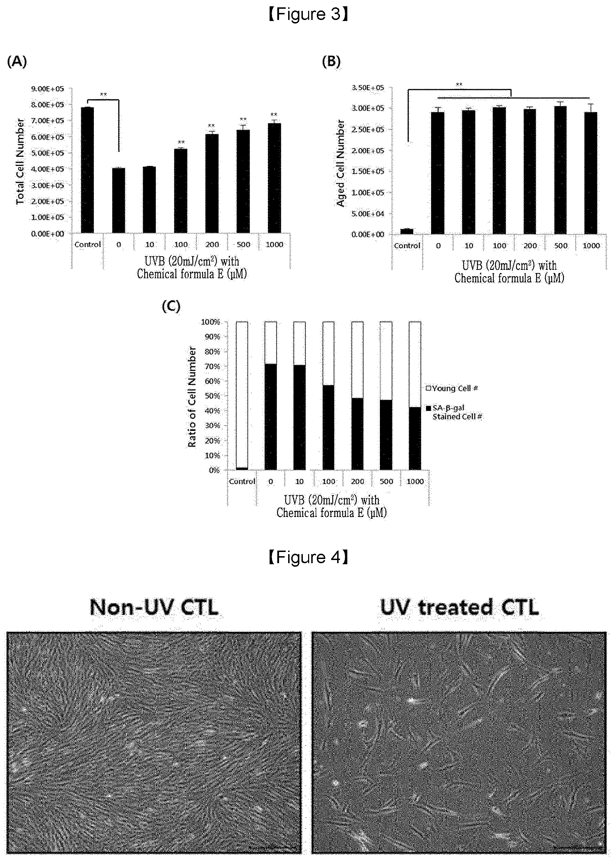

Pigment-forming cells were aged by inducing UVB thereto, and as a result, exhibited morphological changes, and 50 % of the cells were killed due to the sudden aging. Subsequently, the cells were treated with a compound represented by Chemical Formula E (Sigma-Aldrich Co., Ltd.). As a result, depending on a concentration of the compound represented by Chemical Formula E, the number of cells increased again (refer to FIGS. 1 and 2). In addition, when the total number of cells and the number of aged cells were counted to calculate a ratio of the number of the aged cells stained with SA-β-gal to the total number of cells, the ratio of the number of the aged cells to the total number of cells decreased depending on the concentration of the compound represented by Chemical Formula E (refer to FIG. 3).

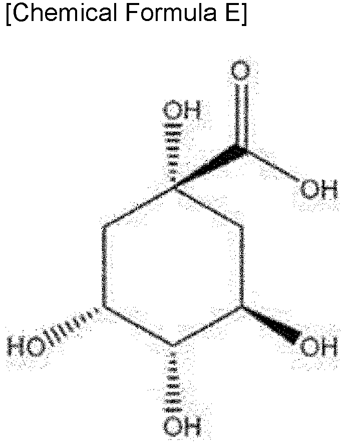

### Experimental Example 2: Change in the number of fibroblast cells after UVB treatment

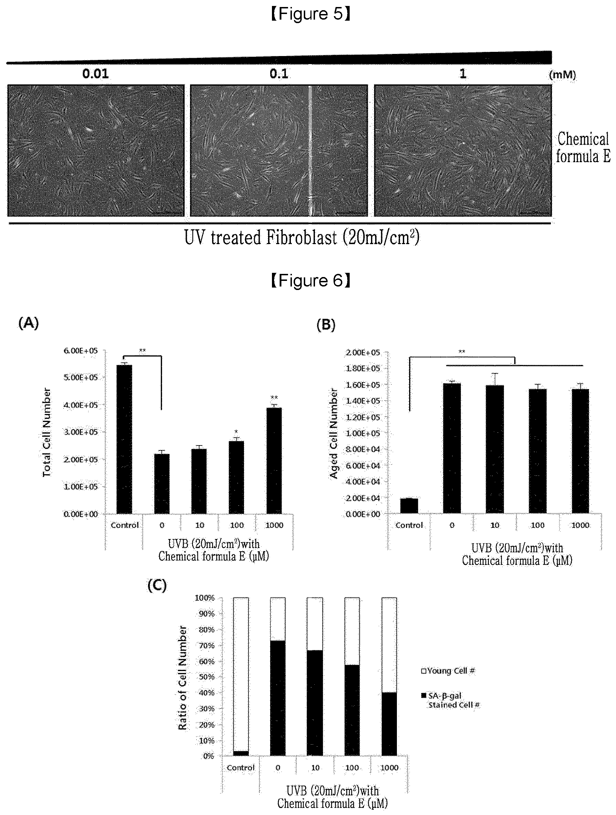

Fibroblast cells were aged by inducing UVB thereto, and as a result, exhibited morphological changes, and about 60 % of the cells were killed due to the sudden aging. Subsequently, the cells were treated with a compound represented by Chemical Formula E. As a result, because of the compound represented by Chemical Formula E, the number of cells increased again depending on the concentration of the compound (refer to FIGS. 4 and 5). In addition, when the total number of cells and the number of aged cells were counted to calculate a ratio of the number of the aged cells stained with SA-β-gal to the total number of cells, the ratio of the number of the aged cells to the total number of cells decreased depending on the concentration of the compound represented by Chemical Formula E (refer to FIG. 6).

## Claims

1. A cosmetic use of a cosmetically acceptable compound represented by Chemical Formula 1, or a salt thereof, for stimulating only the growth of human-derived skin cells that are not stained with SA-β-gal (senescence associated-beta galactosidase): 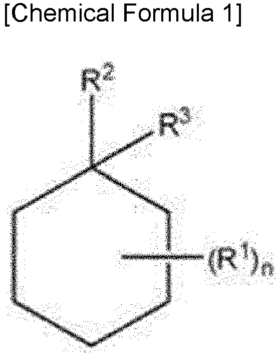 wherein, in Chemical Formula 1,
R¹ to R³ are each independently a hydroxy group or a carboxyl group, and
n is an integer of 1 to 3.

2. The cosmetic use of claim 1, wherein the compound represented by Chemical Formula 1 is represented by Chemical Formula 1-1: 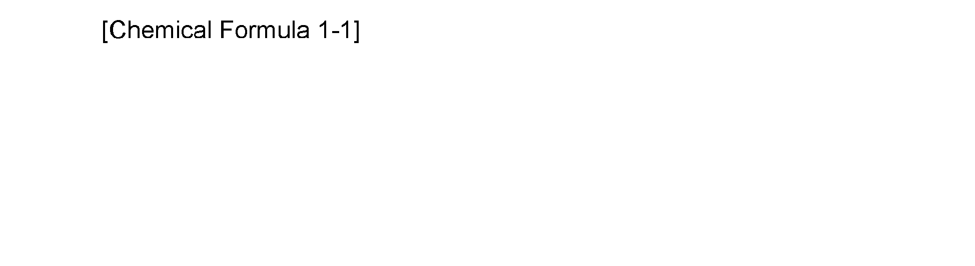 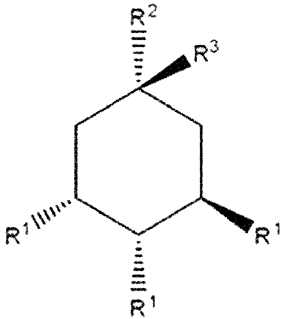 wherein, in Chemical Formula 1-1,
R¹ and R² are each independently a hydroxy group, and
R³ is a carboxyl group.

3. The cosmetic use of claim 1, wherein the compound represented by Chemical Formula 1 is used in an amount of 0.01 wt% to 5 wt% based on the total amount of a composition.

4. The cosmetic use of claim 1, wherein the skin cells are pigment-forming cells.

5. The cosmetic use of claim 4, wherein the compound selectively stimulates growth of only human-derived pigment-forming cells that are not stained with SA-β-gal (senescence associated-beta galactosidase) between human-derived pigment-forming cells that are stained with SA-β-gal (senescence associated-beta galactosidase) and human-derived pigment-forming cells that are not stained with SA-β-gal (senescence associated-beta galactosidase).

6. The cosmetic use of claim 1, wherein the skin cells are fibroblasts.

7. The cosmetic use of claim 6, wherein the compound selectively stimulates growth of only fibroblasts that are not stained with SA-β-gal (senescence associated-beta galactosidase) between human-derived fibroblasts that are stained with SA-β-gal (senescence associated-beta galactosidase) and human-derived fibroblasts (primary fibroblasts; FB) that are not stained with SA-β-gal (senescence associated-beta galactosidase).

8. The cosmetic use of claim 1, wherein
the compound
increases the number of cells only in human-derived young skin cells that cannot be stained with SA-β-gal (senescence associated-beta galactosidase), and
maintains the number of human-derived aged cells that are stained with SA-β-gal (senescence associated-beta galactosidase).

9. The cosmetic use of claim 1, wherein the compound is present in a cosmetic composition.

## Patentansprüche

1. Kosmetische Verwendung einer kosmetisch akzeptablen Verbindung, die durch die chemische Formel 1 dargestellt ist, oder eines Salzes hiervon, zur Stimulierung lediglich des Wachstums der vom Menschen stammenden Hautzellen, die nicht mit SA-β-gal (Seneszenz-assoziierte Beta-Galactosidase) angefärbt werden: 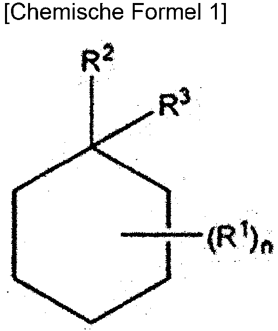 wobei in der chemischen Formel 1
R¹ bis R³ jeweils unabhängig voneinander eine Hydroxygruppe oder eine Carboxylgruppe sind und
n eine ganze Zahl von 1 bis 3 ist.

2. Kosmetische Verwendung nach Anspruch 1, wobei die durch die chemische Formel 1 dargestellte Verbindung durch die chemische Formel 1-1 dargestellt ist: 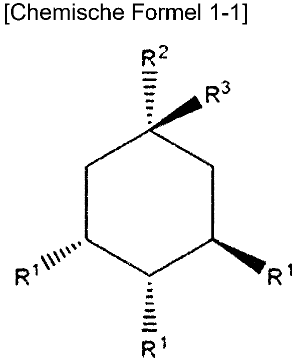 wobei in der chemischen Formel 1-1
R¹ und R² jeweils unabhängig voneinander eine Hydroxygruppe sind und
R³ eine Carboxylgruppe ist.

3. Kosmetische Verwendung nach Anspruch 1, wobei die durch die chemische Formel 1 dargestellte Verbindung in einer Menge von 0,01 Gew.-% bis 5 Gew.-%, bezogen auf die Gesamtmenge einer Zusammensetzung, verwendet wird.

4. Kosmetische Verwendung nach Anspruch 1, wobei die Hautzellen pigmentbildende Zellen sind.

5. Kosmetische Verwendung nach Anspruch 4, wobei die Verbindung selektiv das Wachstum lediglich der vom Menschen stammenden pigmentbildenden Zellen, die nicht mit SA-β-gal (Seneszenz-assoziierte Beta-Galactosidase) angefärbt werden, unter den vom Menschen stammenden pigmentbildenden Zellen, die mit SA-β-gal (Seneszenz-assoziierte Beta-Galactosidase) angefärbt werden, und den vom Menschen stammenden pigmentbildenden Zellen, die nicht mit SA-β-gal (Seneszenz-assoziierte Beta-Galactosidase) angefärbt werden, stimuliert.

6. Kosmetische Verwendung nach Anspruch 1, wobei die Hautzellen Fibroblasten sind.

7. Kosmetische Verwendung nach Anspruch 6, wobei die Verbindung selektiv das Wachstum lediglich von Fibroblasten, die nicht mit SA-β-gal (Seneszenz-assoziierte Beta-Galactosidase) angefärbt werden, unter den vom Menschen stammenden Fibroblasten, die mit SA-β-gal (Seneszenz-assoziierte Beta-Galactosidase) angefärbt werden, und den vom Menschen stammenden Fibroblasten (primäre Fibroblasten; FB), die nicht mit SA-β-gal (Seneszenz-assoziierte Beta-Galactosidase) angefärbt werden, stimuliert.

8. Kosmetische Verwendung nach Anspruch 1, wobei die Verbindung die Anzahl der Zellen lediglich der vom Menschen stammenden jungen Hautzellen erhöht, die nicht mit SA-β-gal (Seneszenz-assoziierte Beta-Galactosidase) angefärbt werden können, und die Anzahl der vom Menschen stammenden gealterten Zellen, die mit SA-β-gal (Seneszenz-assoziierte Beta-Galactosidase) angefärbt werden, aufrechterhält.

9. Kosmetische Verwendung nach Anspruch 1, wobei die Verbindung in einer kosmetischen Zusammensetzung enthalten ist.

## Revendications

1. Utilisation cosmétique d'un composé cosmétiquement acceptable représenté par la Formule Chimique 1, ou d'un sel de celui-ci, pour stimuler uniquement la croissance de cellules cutanées d'origine humaine qui ne sont pas colorées par SA-β-gal (bêta-galactosidase associée à la sénescence) : 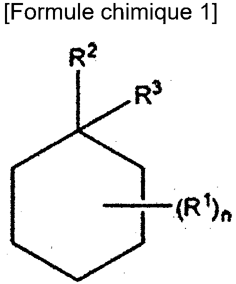 dans laquelle, dans la Formule chimique 1,
R¹ à R³ sont chacun indépendamment un groupe hydroxy ou un groupe carboxyle, et
n est un entier de 1 à 3.

2. Utilisation cosmétique selon la revendication 1, dans laquelle le composé représenté par la Formule chimique 1 est représenté par la Formule chimique 1-1 : 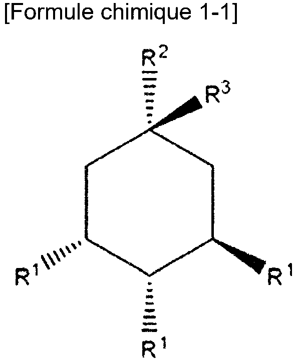 dans laquelle, dans la Formule chimique 1-1,
R¹ et R² sont chacun indépendamment un groupe hydroxy, et
R³ est un groupe carboxyle.

3. Utilisation cosmétique selon la revendication 1, dans laquelle le composé représenté par la Formule chimique 1 est utilisé en une quantité de 0,01 % en poids à 5 % en poids par rapport à la quantité totale d'une composition.

4. Utilisation cosmétique selon la revendication 1, dans laquelle les cellules cutanées sont des cellules formant des pigments.

5. Utilisation cosmétique selon la revendication 4, dans laquelle le composé stimule sélectivement la croissance uniquement des cellules formant des pigments d'origine humaine qui ne sont pas colorées par SA-β-gal (bêta-galactosidase associée à la sénescence), parmi les cellules formant des pigments dérivées de l'humain qui sont colorées par SA-β-gal (bêta-galactosidase associée à la sénescence) et les cellules formant des pigments dérivées de l'humain qui ne sont pas colorées par SA-β-gal (bêta-galactosidase associée à la sénescence).

6. Utilisation cosmétique selon la revendication 1, dans laquelle les cellules cutanées sont des fibroblastes.

7. Utilisation cosmétique selon la revendication 6, dans laquelle le composé stimule sélectivement la croissance uniquement des fibroblastes qui ne sont pas colorés par SA-β-gal (bêta-galactosidase associée à la sénescence), parmi les fibroblastes d'origine humaine qui sont colorés par SA-β-gal (bêta-galactosidase associée à la sénescence) et les fibroblastes d'origine humaine (fibroblastes primaires ; FB) qui ne sont pas colorés par SA-β-gal (bêta-galactosidase associée à la sénescence).

8. Utilisation cosmétique selon la revendication 1, dans laquelle le composé
augmente le nombre de cellules uniquement dans les cellules cutanées jeunes d'origine humaine qui ne peuvent pas être colorées par SA-β-gal (bêta-galactosidase associée à la sénescence), et
maintient le nombre de cellules âgées d'origine humaine qui sont colorées par SA-β-gal (bêta-galactosidase associée à la sénescence).

9. Utilisation cosmétique selon la revendication 1, dans laquelle le composé est présent dans une composition cosmétique.
